# EUROPEAN PATENT APPLICATION

(11) **EP 4 382 522 A1**
(43) Date of publication of application: **12.06.2024**
(21) Application number: 22852015.1
(22) Date of filing: 28.07.2022
(51) Int. Cl.: C07D 401/14, C07D 401/12, C07D 239/42, A61P 29/00, A61P 37/06, A61P 19/02, A61P 1/00, A61P 11/00, A61P 19/08, A61P 17/00, A61P 17/06, A61P 27/02, A61K 31/506, A61K 31/505

(54) **JAK INHIBITOR WITH HIGH ORAL BIOAVAILABILITY**

(30) Priority: 06.08.2021 CN 202110901146
(71) Applicant: Technoderma Medicines Inc., Chengdu, Sichuan 610219 (CN)
(72) Inventor: FANG, Wenkui Ken, Jiaxing, Zhejiang 314000 (CN); LI, Guanqun, Jiaxing, Zhejiang 314000 (CN); CAI, Yuting, Jiaxing, Zhejiang 314000 (CN); PAN, Xiang, Jiaxing, Zhejiang 314000 (CN); ZHU, Wenhao, Jiaxing, Zhejiang 314000 (CN); WANG, Yang, Jiaxing, Zhejiang 314000 (CN); WANG, Zengquan, Jiaxing, Zhejiang 314000 (CN)
(74) Representative: Brann AB
(86) International application number: PCT/CN2022/108488
(87) International publication number: WO 2023/011301

(57) **Abstract**

The present invention provides a JAK inhibitor having high oral bioavailability, characterized in that the JAK inhibitor comprises: a Formula I compound, or a stereoisomer, geometric isomer, tautomer, hydrate, solvate, or pharmaceutically acceptable salt thereof as an active ingredient. The JAK inhibitor with high oral bioavailability provided by the present invention can overcome the problem that existing JAK inhibitors have low oral bioavailability, and involves further modifying a chemical compound so as to change the physicochemical characteristics of one or several small molecule compounds, thus increasing the in-vivo cell absorption performance of said compound(s), greatly improving the bioavailability of the medication and providing new possibilities for drug delivery and utilization for said compound medication.

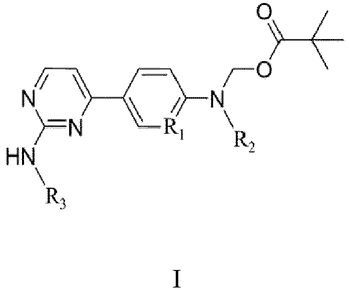

## Description

### CROSS-REFERENCE TO RELATED APPLICATIONS

This application claims priority to Chinese Patent Application No. 202110901146.3 filed on August 06, 2021, which is incorporated herein by reference in its entirety.

### TECHNICAL FIELD

The present disclosure belongs to the field of small molecule compounds, and in particular, relates to a JAK inhibitor with high oral bioavailability, where the JAK inhibitor can be used for prevention or treatment of autoimmune diseases or related inflammatory skin diseases, and has significant advantages especially when administered orally.

### BACKGROUND

Since the launch of Pfizer's JAK3 inhibitor tofacitinib and Incyte's JAK1/JAK2 inhibitor ruxolitinib, JAK inhibitors have gradually become essential therapeutic approaches and a focus for drug development in the treatment of conditions such as allergies, autoimmune-related inflammatory diseases, myeloproliferative diseases (MPD), and graft-versus-host diseases (GVHD).

Currently, eight JAK inhibitors have been globally approved for oral or topical use in the treatment of human diseases. Given the structural similarity and differentiated while overlapping functions among the four subtypes of JAK family members, existing JAK inhibitors predominantly exhibit high efficacy in inhibiting JAK1, JAK2, and JAK3 subtypes. However, these inhibitors encounter challenges in clinical applications, such as suboptimal efficacy and notable side effects. For instance, long-term use of several orally administered JAK inhibitors has gradually revealed safety risks (cancers and infections), prompting the FDA to issue safety (black box) warnings for marketed JAK drugs.

At present, JAK inhibitors face several critical issues that require urgent resolution: 1) the outlook of pan-JAK inhibitors is not promising in terms of oral treatment of non-malignant inflammatory diseases due to the inhibition effects of the pan-JAK inhibitors on more than 3 JAK subtypes, especially the inhibition effect on JAK2, which causes anemia and coagulation disorders; 2) inhibiting 1-2 JAK subtypes, such as JAK1 and TyK2, may enhance safety, but under the condition of specificity, the inhibitory effects are insufficient; 3) JAK inhibitors targeting 1-2 subtypes lack sufficient bioavailability for systemic treatment via oral administration, significantly restricting clinical applications; and 4) small-molecule compounds with high specificity for inhibiting both Tyk2 and JAK1 subtypes are rare, and there is a lack of clinical application data for such compounds in autoimmune diseases.

### SUMMARY

An objective of the present disclosure is to overcome the foregoing shortcomings in the prior art and to provide a JAK inhibitor with high activity, high selectivity and high oral bioavailability.

Specifically, the inventors of the present disclosure have found that the existing JAK inhibitors are suitable for topical application. In animal models of inflammatory skin diseases, such JAK inhibitor medications are passively/actively absorbed into local lesion tissues of the skin, controlling inflammation, treating inflammatory skin diseases, and demonstrating good efficacy. However, these small-molecule JAK inhibitors have extremely low oral bioavailability, making them unsuitable for further development as orally administered medications.

To resolve the problem of low oral bioavailability, the present disclosure alters the physicochemical properties of one or several types of small-molecule compounds by modifying the compounds, thus increasing in vivo cellular absorption performance of the compounds, greatly improving their bioavailability, and providing new possibilities for the administration and usage of these compounds. This innovative approach expands the clinical application pathways for JAK inhibitors with strong inhibition of JAK kinases at both in vitro and in vivo cellular levels, addressing unmet clinical needs in the field of new drug development. The present disclosure is based on the findings mentioned above.

To achieve the foregoing objective, an aspect of the present disclosure provides a JAK inhibitor with high oral bioavailability, where the JAK inhibitor includes a compound of Formula I, or a stereoisomer, geometric isomer, tautomer, hydrate, solvate, or pharmaceutically acceptable salt thereof as an active ingredient: 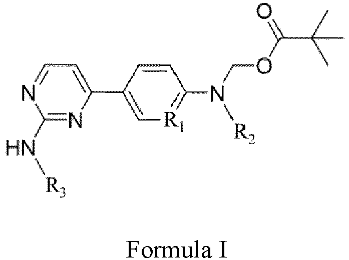 where:
R₁ is CorN;
R₂ is selected from cyano group; and 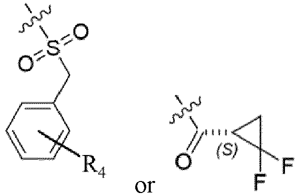 , where R₄ is selected from halogen or a
R₃ is selected from a five- or six-membered aryl or heteroaryl group which is unsubstituted or optionally substituted by at least one of halogen, a C₁-C₃ alkyl group, a hydroxyl group, a C₁-C₃ hydroxyalkyl group, an amino group, an amide group and a C₁-C₃ alkylamide group.

In an embodiment of the present disclosure, R₄ is selected from fluorine or a cyano group.

In an embodiment of the present disclosure, the five- or six-membered aryl or heteroaryl group is selected from a phenyl group, a pyridyl group, a pyrimidyl group, a pyrazolyl group, a pyrrolyl group, an imidazolyl group, a pyrazinyl group or a pyridazinyl group.

In an embodiment of the present disclosure, the C₁-C₃ alkyl group is a methyl group, an ethyl group or a propyl group; the C₁-C₃ hydroxyalkyl group is a hydroxymethyl group, a hydroxyethyl group or a hydroxypropyl group; and the C₁-C₃ alkylamide group is a methylformamide group, a dimethylformamide group, an ethylformamide group or a methylacetamide group.

In an embodiment of the present disclosure, R₃ is selected from any one of:

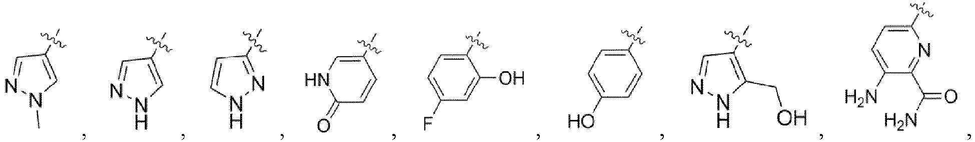

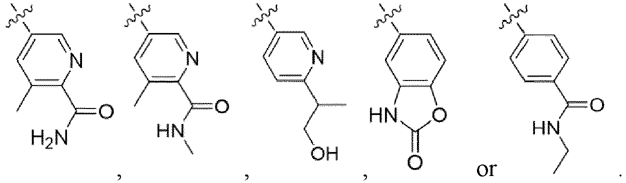

A further aspect of the present disclosure provides use of the compound of the Formula I, or the stereoisomer, geometric isomer, tautomer, hydrate, solvate, or pharmaceutically acceptable salt thereof in preparation of a medication for prevention or treatment of an autoimmune disease or a related inflammatory skin disease.

In an embodiment of the present disclosure, the autoimmune disease is selected from at least one of rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, and uveitis.

In an embodiment of the present disclosure, the related inflammatory skin disease is selected from at least one of psoriasis, autoimmune-related vasculitis, scleroderma, dermatomyositis, acrodermatitis enteropathica, hidradenitis suppurativa, lichen planus, vitiligo, cutaneous lupus erythematosus, and lichen sclerosus et atrophicus.

In an embodiment of the present disclosure, the medication is an orally administered medication.

In an embodiment of the present disclosure, the orally administered medication is in a form of a tablet, a pill, a granule, a capsule, a lozenge or a liquid formulation.

The present disclosure achieves the following effects:

The JAK inhibitor with high oral bioavailability provided by the present disclosure can overcome the existing challenges of low oral bioavailability in JAK inhibitors. Through further compound modification to alter the physicochemical properties of one or several types of small-molecule compounds, the present disclosure enhances the in vivo cellular absorption performance of such compounds, significantly improving the drug's bioavailability. It also offers new possibilities for the administration and usage of such compounds.

Specifically, the JAK inhibitors of the present disclosure achieve improved bioavailability by introducing hydrolyzable metabolic groups to reduce the compound's polarity, increase water solubility, and, by extending the drug's half-life and slowing down drug clearance, significantly enhance the drug's blood concentration. This transformation turns a class of highly effective JAK inhibitors that were previously unsuitable for oral administration into formulations suitable for oral administration, which expands the routes of drug administration, consequently broadening the therapeutic indications for these drugs.

### DETAILED DESCRIPTION

The specific embodiments of the present disclosure will be described in detail hereinafter. It should be understood that the specific embodiments described herein are only intended to illustrate and explain the present disclosure rather than limit the present disclosure.

The endpoints and any value of ranges disclosed herein are not limited to precise ranges or values, and these ranges or values should be understood as including values close to these ranges or values. For numerical ranges, combination can be made among the endpoint values of each range, between the endpoint values of each range and individual point values, and among individual point values to obtain one or more new numerical ranges, and these numerical ranges should be regarded as being specifically disclosed herein.

Before detailed description of the present disclosure, it should be understood that the terms used herein are intended to describe a specific embodiment other than limit the scope of the present disclosure, where the scope of the present disclosure is limited only by the appended claims. To more completely understand the present disclosure described herein, the following terms are used, and the definitions of the terms are shown below. Unless otherwise defined, all technical and scientific terms used herein have the same meanings as those understood by persons of ordinary skills in the art to which the present disclosure belongs.

An aspect of the present disclosure provides a JAK inhibitor with high oral bioavailability, where the JAK inhibitor includes a compound of Formula I, or a stereoisomer, geometric isomer, tautomer, hydrate, solvate, or pharmaceutically acceptable salt thereof as an active ingredient, 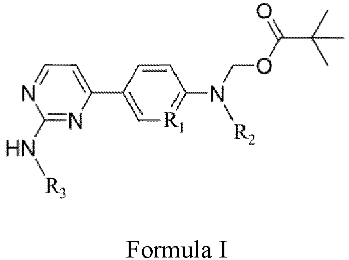 where:
R₁ is CorN;
R₂ is selected from 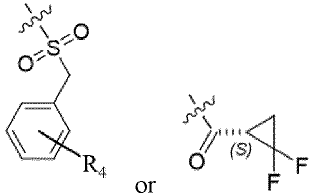 , where R₄ is selected from halogen (for example, fluorine, chlorine or bromine, in particular, fluorine) or a cyano group; and
R₃ is selected from a five- or six-membered aryl or heteroaryl group which is unsubstituted or optionally substituted by at least one of halogen, a C₁-C₃ alkyl group, a hydroxyl group, a C₁-C₃ hydroxyalkyl group, an amino group, an amide group and a C₁-C₃ alkylamide group.

In the present disclosure, the five- or six-membered aryl or heteroaryl group which is unsubstituted or substituted by a substituent may be various five- or six-membered aryl or heteroaryl groups well known in the art. The heteroatom(s) in the heteroaryl group can include N, O, S or the like, as known to those skilled in the art, and preferably N atom. The number of the heteroatoms can be, for example, 1, 2, or 3. In a preferred embodiment of the present disclosure, the five- or six-membered aryl or heteroaryl group may be selected from, for example, a phenyl group, a pyridyl group, a pyrimidyl group, a pyrazolyl group, a pyrrolyl group, an imidazolyl group, a pyrazinyl group or a pyridazinyl group, but is not limited thereto.

For the foregoing substituents, the C₁-C₃ alkyl group can be, for example, a methyl group, an ethyl group or a propyl group; the C₁-C₃ hydroxyalkyl group can be, for example, a hydroxymethyl group, a hydroxyethyl group or a hydroxypropyl group; and the C₁-C₃ alkylamide group can be, for example, a methylformamide group, a dimethylformamide group, an ethylformamide group or a methylacetamide group, but is not limited thereto.

Further, in a more preferred embodiment of the present disclosure, R₃ can be selected from any one of:

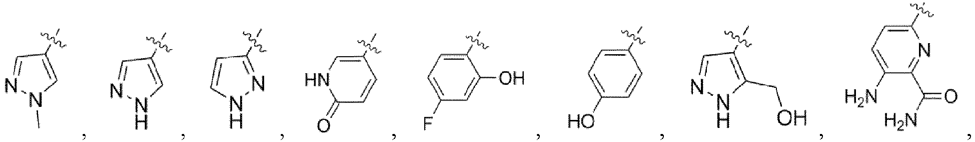

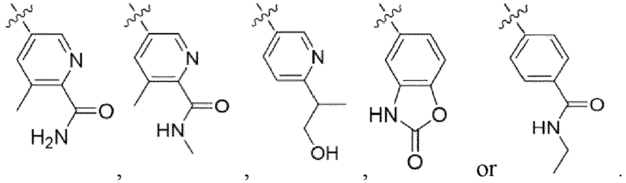

The term "pharmaceutically acceptable" as used herein refers to a substance which does not affect the biological activity or properties of the compound of the present disclosure, and is relatively non-toxic, that is, the substance can be administered to a subject without causing an adverse biological reaction or interacting with any ingredient contained in the composition in an adverse manner. In the present disclosure, "pharmaceutically acceptable salts" may include inorganic salts and organic salts, where the organic salts may include but not limited to ammonium, lithium, sodium, potassium, cesium, calcium, magnesium, copper, aluminum, zinc, barium or quaternary ammonium salts, and the inorganic salts may include but not limited to arginine, tert-butylamine, dimethylamine, diethanolamine, ethanolamine, ethylenediamine, imidazole, lysine, methylamine, pyridine, pyridinecarboxylate, piperazine, triethylamine, triethanolamine, trimethylamine, or urea salts.

Another aspect of the present disclosure further provides use of the compound of the Formula I, or the stereoisomer, geometric isomer, tautomer, hydrate, solvate, or pharmaceutically acceptable salt thereof in preparation of a medication for prevention or treatment of an autoimmune disease or a related inflammatory skin disease.

The term "treatment" as used herein refers to any administration of a therapeutic agent according to a therapeutic regimen, where the therapeutic regimen achieves desired effects, that is, effects of partially or completely relieving, improving, remitting, inhibiting, delaying, and reducing severity and/or incidence of one or more symptoms or conditions of a particular disease, disorder, and/or condition; and in some embodiments, administration of a therapeutic agent according to a therapeutic regimen is associated with achievement of desired effects. Such treatment may be prescribed for a subject who does not have a relevant disease, disorder, and/or symptom, and/or a subject having only an early sign of the disease, disorder and/or condition. Alternatively or additionally, such treatment may be prescribed for a subject having one or more established signs of the relevant disease, disorder, and/or condition. In some embodiments, the treatment may be prescribed for a subject who has been diagnosed with the relevant disease, disorder, and/or condition. In some embodiments, the treatment may be prescribed for a subject for whom one or more predisposing factors have been identified, where the predisposing factors are statistically associated with an increased risk of developing the relevant disease, disorder, and/or condition.

According to the present disclosure, the medications prepared for the foregoing use may include an effective amount of the compound of Formula I of the present disclosure, and a pharmaceutically acceptable excipient, carrier, or diluent.

The terms "effective amount", "therapeutically effective amount", or "pharmaceutically effective amount" as used herein refer to a dose of a therapeutic agent that confers a therapeutic effect on a subject being treated at an appropriate benefit-risk ratio applicable to any medication. Such a therapeutic effect may be objective (that is, the therapeutic effect may be measured through a specific test or marker) or subjective (that is, the effect is indicated or felt by the subject). In some embodiments, the "therapeutically effective amount" refers to a dose of a therapeutic agent or a composition for effectively treating, improving, or preventing (for example, delaying onset) of a related disease or symptom, and/or conferring a detectable therapeutic or prophylactic effect by improving a symptom associated with a disease, preventing or delaying the onset of the disease, and/or also reducing severity or frequency of the symptom.

Those skilled in the art will understand that the therapeutically effective amount of the compound of Formula I to be administered will vary depending on the nature and severity of the subject and the disease, the physical condition of the subject, the treatment regimen (for example, whether a second therapeutic agent is used), and the selected route of administration. An appropriate dosage can be readily determined by those skilled in the art. Additionally, the optimal amount and intervals of individual doses of the drug will be determined by the nature and severity of the condition being treated, the form, the route, and the location of administration, as well as the age and condition of the specific subject being treated. The physician will ultimately determine the appropriate dosage for administration. The dosage may be repeated multiple times as needed. If side effects occur, the amount and/or frequency of the dose may be changed or reduced based on normal clinical practice.

In the present disclosure, the "pharmaceutically acceptable excipient, carrier, or diluent" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or the like, that are permitted by relevant government authorities for human or animal use.

Furthermore, according to the present invention, the medications produced in the aforementioned uses, in addition to containing the compound of Formula (I) of the present invention as an active ingredient, may also include other agents for preventing or treating autoimmune diseases and immunologically related inflammatory skin conditions as another effective component. Examples of such pharmaceutical agents include, but are not limited to, vitamin D derivatives, vitamin A derivatives, glucocorticoid, calcineurin inhibitors, or non-steroidal anti-inflammatory drugs. When the medication contains a plurality of effective ingredients, the effective ingredients may be administered simultaneously, sequentially, or separately at the discretion of the physician.

In addition, the compound of the Formula I according to the present disclosure can be administered to a patient through various routes, such as oral, transdermal, subcutaneous, intranasal, intravenous, intramuscular, intrathecal, regional, or topical (for example, mucosa) route. The most appropriate route of administration in any given situation depends on the subject and the nature and severity of the disease, the physical conditions of the subject, and the like. In an embodiment of the present disclosure, the medication prepared with the compound of the Formula I of the present disclosure may be administered orally. In this case, the orally administered medication may be in a form of a tablet, a pill, a granule, a capsule, a lozenge or a liquid formulation.

Through the inventor's research, it has been discovered that the compound of Formula (I) of the present invention or the medications derived from it exhibit outstanding effectiveness when used for the prevention or treatment of JAK-related autoimmune diseases and related inflammatory skin diseases, particularly even when administered orally. Specifically, the autoimmune diseases include but are not limited to rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, uveitis, and the like; and the related inflammatory skin diseases are selected from psoriasis, autoimmune-related vasculitis, scleroderma, dermatomyositis, acrodermatitis enteropathica, hidradenitis suppurativa, lichen planus, vitiligo, cutaneous lupus erythematosus, lichen sclerosus et atrophicus, and the like.

The effects of the specific compounds of the present disclosure will be described in detail below.

### Examples

### Example 1: General Method for Synthesizing Compound 1 (TDM-181055)

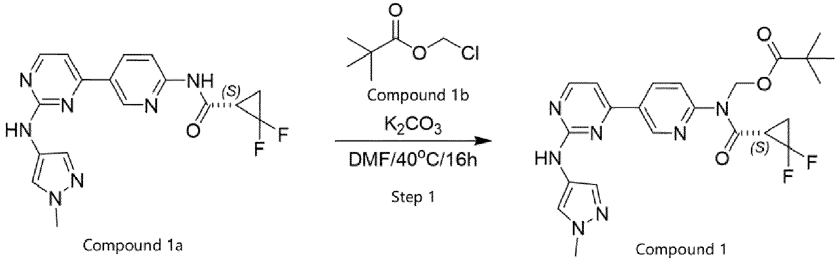

### Step 1: Preparation of Compound 1 (TDM-181055) ((S)-(2,2-difluoro-N-(5-(2-(((1-methyl-1H-pyrazol-4-yl)amino)pyrimidin-4-yl)pyridin-2-yl)cyclopropane-1-carboxamido)methyl pivalate)

Potassium carbonate (372 mg, 2.692 mmol) and compound 1b (608 mg, 4.039 mmol) were added to a solution of compound 1a (500 mg, 1.346 mmol) in N,N-dimethylformamide (15 mL), and the mixture was purged with argon several times, heated to 40°C, and stirred overnight. Then the mixture was concentrated under reduced pressure, water was added to the residue, the solid was collected by filtration, and the solid was purified via silica gel chromatography (dichloromethane: dichloromethane containing 10% methanol = 0%-30%), to obtain a yellow solid product (TDM-181055, compound 1, 19.8 mg, yield: 3%). LCMS [M+1]⁺ = 486.2.

¹H NMR (400 MHz, DMSO) δ 9.60 (s, 1H), 9.24 (d, *J* = 2.1 Hz, 1H), 8.63 (d, *J* = 7.3 Hz, 1H), 8.55 (d, *J* = 5.1 Hz, 1H), 7.93 (s, 1H), 7.63 (d, *J* = 8.5 Hz, 1H), 7.56 (s, 1H), 7.41 (d, J= 5.2 Hz, 1H), 6.03 - 5.92 (m, 2H), 3.83 (s, 3H), 3.04 (s, 1H), 2.17 - 1.88 (m, 2H), 1.10 (s, 9H).

### Example 2: General Method for Synthesizing Compound 2 (TDM-181065)

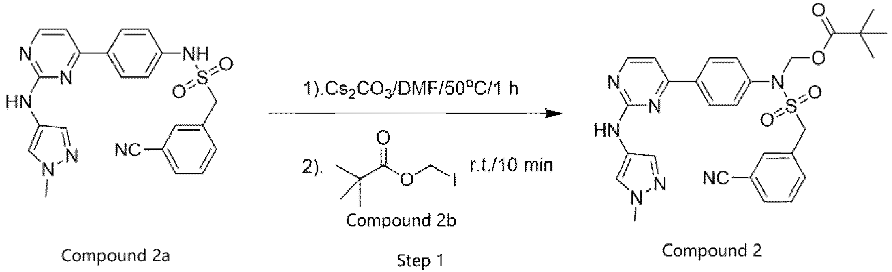

### Step 1: Preparation of Compound 2 (TDM-181065) (((1-(3-cyanophenyl)-N-(4-(2-(((1-methyl-1H-pyrazol-4-yl)amino)pyrimidin-4-yl)phenyl)methyl)sulfonamido)methyl pivalate)

Cesium carbonate (219.4 mg, 0.673 mmol) was added to a solution of compound 2a (150 mg, 0.337 mmol) in N,N-dimethylformamide (10 mL), and the reaction solution was heated to 50°C and stirred for 1 hour. The reaction solution was naturally cooled to room temperature, and then compound 2b (122.2 mg, 0.505 mmol) was added. The reactant solution was stirred for 10 minutes at room temperature, LCMS[M+H]⁺=560 was detected, indicating the completion of the reaction. Post-treatment: The reaction solution was added into 70 mL of water, the aqueous phase was extracted three times with ethyl acetate EA (3*150 mL), the organic phases were combined and rinsed with saturated salt water, dried with anhydrous sodium sulfate, and suction filtered, the filtrate was dried, and the obtained crude product was purified via column chromatography [eluent: (D/M=10:1)/DCM=0%-15%], to obtain the target compound (TDM-181065, compound 2, 60 mg, yield: 31.8%) as a yellow solid. LCMS [M+1]⁺ = 560.2.

¹H NMR (400 MHz, DMSO) δ 9.54 (s, 1H), 8.51 (d, J = 5.1 Hz, 1H), 8.18 (d, J = 8.6 Hz, 2H), 7.94 - 7.86 (m, 3H), 7.79 (d, J = 8.1 Hz, 1H), 7.63 (t, J = 7.8 Hz, 1H), 7.58 (s, 1H), 7.50 (d, J = 8.3 Hz, 2H), 7.31 (d, J = 5.2 Hz, 1H), 5.63 (s, 2H), 4.86 (s, 2H), 3.84 (s, 3H), 1.17 (s, 9H).

### Example 3: General Method for Synthesizing Compound 3 (TDM-181058)

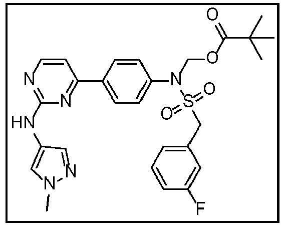

A yellow solid compound (TDM-181058, compound 3, 90.5 mg, yield: 41.13%) was obtained using a method similar to that in Example 2. LCMS [M+1]⁺ = 553.3.

¹H NMR (400 MHz, DMSO) δ 9.53 (s, 1H), 8.51 (d, J = 5.1 Hz, 1H), 8.18 (d, J = 8.6 Hz, 2H), 7.91 (s, 1H), 7.57 (s, 1H), 7.53 - 7.41 (m, 3H), 7.27 (ddd, J = 17.1, 11.2, 3.5 Hz, 4H), 5.60 (s, 2H), 4.79 (s, 2H), 3.83 (s, 3H), 1.17 (s, 9H).

### Example 4: General Method for Synthesizing Compound 4 (TDM-181059)

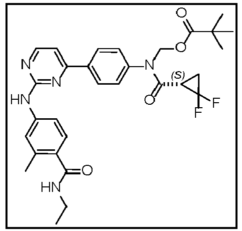

An off-white solid (TDM-181059, compound 4, 67 mg, yield: 23%) was obtained using a method similar to that in Example 2. LCMS [M+1]⁺ = 566.3.

¹H NMR (400 MHz, DMSO) δ 9.81 (s, 1H), 8.62 (d, J = 5.2 Hz, 1H), 8.32 (d, J = 7.4 Hz, 2H), 8.09 (t, J = 5.6 Hz, 1H), 7.71 (d, J = 6.6 Hz, 2H), 7.51 (d, J = 5.2 Hz, 3H), 7.32 (d, J = 9.0 Hz, 1H), 5.74 (d, J = 10.4 Hz, 2H), 3.28 - 3.18 (m, 2H), 2.55 - 2.52 (m, 1H), 2.37 (s, 3H), 2.03 (dt, J = 13.3, 6.6 Hz, 1H), 1.87 (s, 1H), 1.19 - 1.06 (m, 12H).

### Example 5: General Method for Synthesizing Compound 5 (TDM-181060)

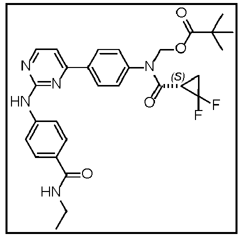

An off-white solid (TDM-181060, compound 5, 89.4 mg, yield: 22%) was obtained using a method similar to that in Example 2. LCMS [M+1]⁺ = 552.2.

¹H NMR (400 MHz, DMSO) δ 10.00 (s, 1H), 8.65 (d, J = 5.2 Hz, 1H), 8.41 - 8.24 (m, 3H), 7.92 (d, J = 8.9 Hz, 2H), 7.83 (d, J = 8.9 Hz, 2H), 7.59 - 7.37 (m, 3H), 5.75 (d, J = 10.4 Hz, 2H), 3.31 - 3.24 (m, 2H), 2.52 (d, J = 1.9 Hz, 1H), 2.13 - 1.78 (m, 2H), 1.19 - 1.06 (m, 12H).

### Comparative Example 1: General Method for Synthesizing Compound 6 (TDM-180935)

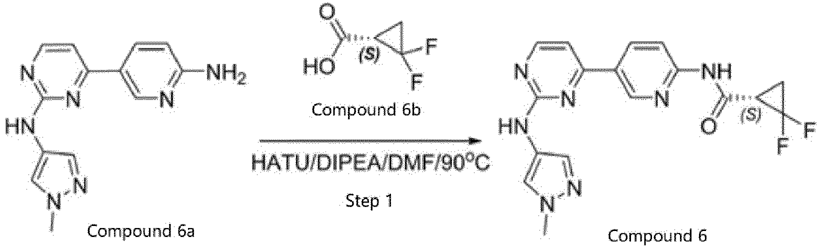

### Step 1: Preparation of Compound 6 (TDM-180935)

N,N-diisopropylethylamine (72.9 mg, 0.564 mmol) was added to a solution of compound 6a (namely, 4-(6-aminopyridin-3-yl)-N-(1-methyl-1H-pyrazol-4-yl)pyrimidin-2-amine) (80 mg, 0.299 mmol) in N,N- dimethylformamide (5 mL) at room temperature, the mixture was stirred for 5 minutes, and then 2-(7-benzotriazole oxide)-N,N,N',N'-tetramethylurea hexafluorophosphate (170 mg, 0.449 mmol) and compound 6b (namely, (S)-2,2-difluorocyclopropane-1-carboxylic acid) (54 mg, 0.449 mmol) were added into the mixture. The mixture was heated to 90°C and stirred for 16 hours. The mixture was concentrated under reduced pressure to remove some solvent, water was added to the residue, and the solution was extracted with ethyl acetate (60 mL*3). The organic layer was washed with saturated brine (50 mL*5), and dried over anhydrous sodium sulfate. The filtrate was concentrated under reduced pressure, and the residue was purified via formic acid to obtain a yellow solid compound, namely, (S)-2,2-difluoro-N-(5-(2-((1-methyl-1H-pyrazol-4-yl)amino)pyrimidin-4-yl)pyridin-2-yl)cycloprop-ane-1-carboxamide (compound 6, 21.6 mg, yield: 19.5%). LCMS [M+1]⁺ = 372.1.

¹H NMR (400 MHz, DMSO-d6) δ 11.29 (s, 1H), 9.54 (s, 1H), 9.11 (d, J = 2.0 Hz, 1H), 8.51 (dd, J = 14.3, 6.9 Hz, 2H), 8.22 (d, J = 8.7 Hz, 1H), 7.93 (s, 1H), 7.54 (s, 1H), 7.34 (d, J = 5.2 Hz, 1H), 3.83 (s, 3H), 3.04 (dd, J = 22.4, 9.9 Hz, 1H), 2.05 (dt, J = 11.8, 8.0 Hz, 2H).

### Comparative Example 2: General Method for Synthesizing Compound 7 (TDM-180958)

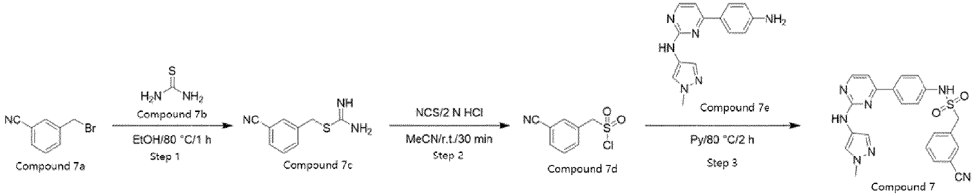

### Step 1: Preparation of Compound 7c (3-cyanobenzylaminohiocarbamate)

Compound 7b (690 mg, 9.08 mmol) was added to a solution of compound 7a (1.78g, 9.08 mmol) in ethanol (13 mL), the reaction solution was heated to 80 °C and stirred for 1 hour, and LCMS[M+H]+=192 was detected, indicating the completion of the reaction. Post-treatment: The reactant solution was concentrated to dryness, to obtain a white target compound (compound 3c, 1.7g, yield: 97.7%), where LCMS [M+1]⁺=192.

### Step 2: Preparation of Compound 7d ((3-cyanophenyl)methanesulfonyl chloride)

2N hydrochloric acid solution (2.5 mL) and compound 7c (1.74g, 9.08 mmol) were added to a solution of N-chlorosuccinimide (4.85g, 36.32 mmol) in acetonitrile (20 mL), and the reaction solution was stirred at room temperature for 30 minutes. Post-treatment: After the reaction ended, the reaction solution was concentrated to remove acetonitrile, water (15 mL) was added, a white solid precipitated, the reaction solution was filtered, and the solid was dried with an oil pump to obtain a white target compound (compound 7d, 1.776g, yield: 90.7%).

### Step 3: Preparation of Compound 7 (TDM-180958)

Compound 7d (1g, 4.64 mmol) was added to a solution of compound 7e (500 mg, 1.878 mmol) in anhydrous pyridine (20 mL), and the reaction solution was heated to 80°C for reaction for 30 minutes, and LCMS[M+H]⁺=446 was detected, indicating the completion of the reaction. Post-treatment: The reaction solution was concentrated to dryness, the crude product was subjected to column chromatography [eluent: (D:M=10:1)/DCM= 0%-50%], the crude product was slurried with DCM/MeOH=30/1 to obtain a yellow solid which was purified via preparative HPLC to obtain a yellow target compound, namely, 1-(3-cyanophenyl)-N-(4-(2-(((1-methyl-1H-pyrazol-4-yl)amino)pyrimidin-4-yl)phenyl)methylsulfonamide (compound 7, 155.4 mg, yield: 18.6%).

¹H NMR (400 MHz, DMSO) δ 10.27 (s, 1H), 9.46 (s, 1H), 8.46 (d, J = 5.2 Hz, 1H), 8.12 (d, J = 8.7 Hz, 2H), 7.93 (s, 1H), 7.84 (dt, J = 7.2, 1.6 Hz, 1H), 7.71 (s, 1H), 7.61 (ddd, J = 17.3, 10.8, 4.8 Hz, 3H), 7.32 (d, J = 8.6 Hz, 2H), 7.24 (d, J = 5.2 Hz, 1H), 4.71 (s, 2H), 3.85 (s, 3H). LCMS[M+H]⁺ = 446.

### Comparative Example 3: General Method for Synthesizing Compound 8 (TDM-180945)

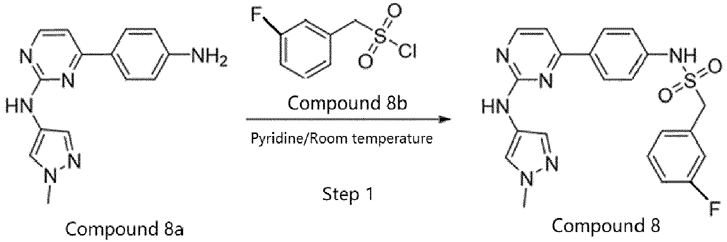

### Step 1: Preparation of Compound 8 (TDM-180945)

Compound 8b (65.8 mg, 0.315 mmol) was added to a solution of the compound 8a (60 mg, 0.225 mmol) in pyridine (5 mL) at room temperature, and the mixture was heated to 70°C and stirred for 6 hours. Then the mixture was concentrated under reduced pressure, and the residue was purified via silica gel chromatography (dichloromethane: dichloromethane containing 10% methanol = 70: 30) and formic acid, to obtain a yellow target solid compound 8, TDM-180945, namely, 1-(3-fluorophenyl)-N-(4-(2-(((1-methyl-1H-pyrazol-4-yl)amino)pyrimidin-4-yl)phenyl)methylsulfonamide (26.1 mg, yield: 19.8%).

¹H NMR (400 MHz, DMSO-d6) δ10.25 (s, 1H), 9.45 (s, 1H), 8.45 (d, J = 5.2 Hz, 1H), 8.12 (d, J = 8.6 Hz, 2H), 7.93 (s, 1H), 7.56 (s, 1H), 7.40 (dd, J = 14.3, 7.6 Hz, 1H), 7.32 (d, J = 8.6 Hz, 2H), 7.26 - 7.15 (m, 2H), 7.11 (d, J = 7.3 Hz, 2H), 4.63 (s, 2H), 3.84 (s, 3H). LCMS [M+1]⁺ = 439.2.

### Comparative Example 4: General Method for Synthesizing Compound 9 (TDM-180977)

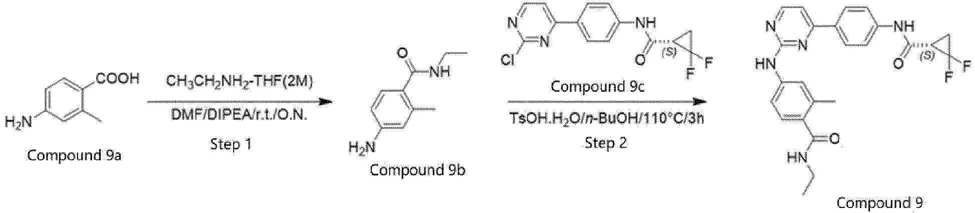

### Step 1: Preparation of Compound 9b (4-amino-N-ethyl-2-methylbenzamide)

2-(7-azabenzotriazole)-N,N,N',N'-tetramethylurea hexafluorophosphate (5.4 g, 14.289 mmol) and N,N-diisopropylethylamine (3.8 g, 29.775 mmol) were added to a solution of compound 9a (1.8 g, 11.91 mmol) in N,N-dimethylformamide (80 mL), the mixture was stirred for 5 minutes, then a solution of ethylamine in tetrahydrofuran (2M) (9 mL, 18 mmol) was added, and the mixture was stirred overnight at room temperature. The mixture was concentrated under reduced pressure to remove some solvent, water was added to the residue, the residue was extracted with ethyl acetate (100 mL*3), the organic phases were combined, rinsed with water (150 mL*3) and saturated brine (150 mL), and dried over sodium sulfate, and the filtrate was concentrated under reduced pressure and purified by silica gel chromatography (petroleum ether/ethyl acetate=0%-50%) to obtain the target compound (compound 9b, 1.32g, yield: 62.2%) as a yellow oil. LCMS [M+1]⁺=179.

### Step 2: Preparation of Compound 9 ((S)-4-((4-(4-(4-(2,2-difluorocyclopropane-1-carboxamido)phenyl)pyrimidin-2-yl)amino)-N-et-hyl-2-methylbenzamide)

Compound 9b (92 mg, 0.517 mmol) and p-toluenesulfonic acid monohydrate (98 mg, 0.517 mmol) were added to a solution of compound 9c (80 mg, 0.258 mmol) in n-butanol (8 mL). The resulting mixture was heated to 110°C and stirred for 3 hours. After the reaction ended, the mixture was concentrated under reduced pressure, and the residue was purified via preparative HPLC (formic acid), to obtain a target compound TDM-180977 (compound 9, 19.4 mg, yield: 13.3%) as a white solid. LCMS[M+H]⁺=425.2.

¹H NMR (400 MHz, DMSO) δ10.70 (s, 1H), 9.72 (s, 1H), 8.54 (d, J = 5.3 Hz, 1H), 8.18 (d, J = 8.8 Hz, 2H), 8.08 (d, J = 5.6 Hz, 1H), 7.81 - 7.67 (m, 4H), 7.40 (d, J = 5.3 Hz, 1H), 7.32 (d, J = 8.4 Hz, 1H), 3.28 - 3.17 (m, 2H), 2.86 (ddd, J = 13.6, 10.8, 8.1 Hz, 1H), 2.37 (s, 3H), 2.14-1.93 (m, 2H), 1.11 (t, J = 7.2 Hz, 3H).

### Comparative Example 5: General Method for Synthesizing Compound 10 (TDM-180972)

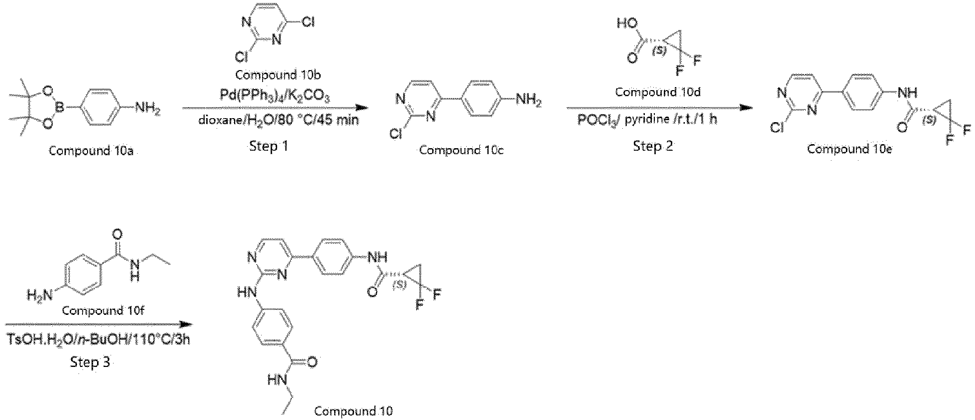

### Step 1: Preparation of Compound 10c (4-(2-chloropyrimidin-4-yl)aniline)

Compound 10a (2g, 9.129 mmol), compound 10b (1.36g, 9.129 mmol), tetrakis(triphenylphosphine)palladium (527g, 0.45 mmol), potassium carbonate (2.5g, 18.258 mmol), dioxane (20 mL), and water (20 mL) were added into a three-necked flask, and nitrogen displacement was performed several times, and then the mixture was heated to 80°C and stirred for 45 minutes. After the reaction ended, the reactants were concentrated under reduced pressure, and the residue was purified by silica gel chromatography [petroleum ether/ethyl acetate = 0%-60%] to obtain the target compound (compound 10c, 394 mg, yield: 21%) as a pale yellow solid. LCMS [M+1]⁺=206.

### Step 2: Preparation of Compound 10e ((S)-N-(4-(2-chloropyrimidin-4-yl)phenyl)-2,2-difluorocyclopropane-1-carboxamide)

Compound 10c (300 mg, 1.459 mmol) and compound 10d (187 mg, 1.531 mmol) were added to a three-necked flask, nitrogen displacement was performed to the mixture several times, and then pyridine (10 mL) and phosphorus oxychloride (335.6 mg , 2.189 mmol) were added at 0°C. The mixture was stirred at room temperature for 1 hour, and then concentrated under reduced pressure, the residue was extracted with ethyl acetate (30 mL*3), organic layers were combined, rinsed with water (50 mL*3) and saturated brine (50 mL*2), and dried over sodium sulfate, the filtrate was concentrated under reduced pressure, and the residue was purified by silica gel chromatography (petroleum ether/ethyl acetate = 0%-12%) to obtain the target compound (compound 10e, 327.7 mg, yield: 72.5%) as a yellow solid. LCMS[M+H]⁺=310.

### Step 3: Preparation of compound 1 ((S)-4-((4-(4-(4-(2,2-difluorocyclopropane-1-carboxamido)phenyl)pyrimidin-2-yl)amino)-N-et-hylbenzamide)

Compound 1f (85 mg, 0.517 mmol) and p-toluenesulfonic acid monohydrate (98 mg, 0.517 mmol) were added to a solution of compound 10e (80 mg, 0.258 mmol) in n-butanol (8 mL). The resulting mixture was heated to 110°C and stirred for 3 hours. After the reaction ended, the mixture was concentrated under reduced pressure, and the residue was purified via preparative HPLC (formic acid), to obtain the target compound TDM-180972 (compound 10, 19.7 mg, yield: 17.5%) as a yellow solid. LCMS[M+H]⁺=438.2.

¹H NMR (400 MHz, DMSO) δ 10.70 (s, 1H), 9.91 (s, 1H), 8.57 (d, J = 5.3 Hz, 1H), 8.28 (t, J = 5.5 Hz, 1H), 8.19 (d, J = 8.8 Hz, 2H), 7.92 (d, J = 8.9 Hz, 2H), 7.83 (d, J = 8.9 Hz, 2H), 7.78 (d, J = 8.8 Hz, 2H), 7.44 (d, J = 5.3 Hz, 1H), 3.28 (dt, J = 12.7, 6.4 Hz, 2H), 2.86 (ddd, J = 13.6, 10.8, 8.0 Hz, 1H), 2.12 - 1.94 (m, 2H), 1.13 (t, J = 7.2 Hz, 3H).

### Biological Description of Oral PK in Rats:

To verify whether compounds 1 to 5 in the examples of the present disclosure have significantly improved oral bioavailability compared to compounds 6 to 10 having similar structures in comparative examples, pharmacokinetic (PK) tests were conducted on compounds 1-10 in Sprague Dawley (SD) rats (about 6-8 weeks old, weighing approximately 250-300 grams) with a single oral dose, so as to compare the PK parameters of compounds 1-10. In brief, the compounds were prepared in a solvent [DMA: 30% Solutol HS 15: Saline = 10: 10: 80 (v/v/v)] to form clear solution formulations having a concentration of 1 mg/mL. Each test compound was administered to 3 rats (n=3) in single oral gavage (5 mg/kg). Approximately 150 µL of whole blood was taken from a subclavian vein of each experimental rat before administration and at 0.25, 0.5, 1, 2, 4, 8, 12, and 24 hours after administration, respectively, and was collected into an Eppendorf tube containing EDTA-K2, then 100 µL of the whole blood was added to an Eppendorf tube containing 300 µL of cyanomethane (ACN), mixed and then centrifuged to separate the plasma, and after it was ensured that there was no hemolysis in the samples, the plasma samples were stored in a freezer at -90°C to -60°C for analyses of drug concentrations in the samples via LC-MS/MS. No adverse reactions to any of the test compounds were observed in the rats in all the oral PK tests.

Concentrations of compounds in the plasma samples were tested and analyzed via LC-MS/MS system (API4000: LC-MS-MS-001), 10 µL of the sample was mixed with 100 µL of ACN containing a standard, then 110 µL of water was added and the mixture was oscillated, and finally, 2 µL of the mixed solution was injected into the LC-MS/MS system.

The test results are as follows:
(1) For the compound in Comparative Example 1: Cₘₐₓ=116 ng/mL, and AUC₀₋ₜ=891 ng h/mL; and after the compound was further synthesized into the compound in Example 1, Cₘₐₓ=668 ng/mL, and AUC₀₋ₜ=1670 ng·h/mL.
(2) For the compound in Comparative Example 2: Cₘₐₓ=20.2 ng/mL, and AUC₀₋ₜ=47.4 ng h/mL; and after the compound was further synthesized into the compound in Example 2, Cₘₐₓ=791 ng/mL, and AUC₀₋ₜ=1580 ng h/mL.
(3) For the compound in Comparative Example 3: Cₘₐₓ=85.5 ng/mL, and AUC₀₋ₜ=381 ng h/mL; and after the compound was further synthesized into the compound in Example 3, Cₘₐₓ=1860 ng/mL, and AUC₀₋ₜ=2890 ng h/mL.
(4) For the compound in Comparative Example 4: Cₘₐₓ=5.02 ng/mL, and AUC₀₋ₜ=30.7 ng h/mL; and after the compound was further synthesized into the compound in Example 4, Cₘₐₓ=635 ng/mL, and AUC₀₋ₜ=3 090 ng h/mL.
(5) For the compound in Comparative Example 5: Cₘₐₓ=16.5 ng/mL, and AUC₀₋ₜ=141 ng h/mL; and after the compound was further synthesized into the compound in Example 5, Cₘₐₓ=689 ng/mL, and AUC₀₋ₜ=2710 ng·h/mL.

According to the biological data of oral PK in rats, it can be seen that after further synthesis, systemic exposure of the compound is significantly increased, demonstrated by a 5-120 times increase in peak plasma drug concentration and a 2-100 times increase in AUC, as compared to the original drug, which proves that the oral bioavailability of the compound in the examples of the present disclosure is significantly improved and the in vivo clearance rate is significantly reduced.

The preferred embodiments of the present disclosure have been described in detail above. However, the present disclosure is not limited to the specific details in the above embodiments. Within the scope of the technical concept of the present disclosure, many simple modifications can be made to the technical solution of the present disclosure, all of which fall within the claimed scope of the present disclosure.

Furthermore, it should be noted that various specific technical features described in the above specific embodiments can be combined in any suitable way without contradiction. In order to avoid unnecessary repetition, the present disclosure will not explain various possible combinations separately.

Furthermore, any combination can be made among various embodiments of the present disclosure, as long as they do not violate the spirit of the present disclosure, and should also be considered as part of the disclosed content of the present disclosure.

## Claims

1. A JAK inhibitor with high oral bioavailability, wherein the JAK inhibitor comprises a compound of Formula I, or a stereoisomer, geometric isomer, tautomer, hydrate, solvate, or pharmaceutically acceptable salt thereof, as an active ingredient, 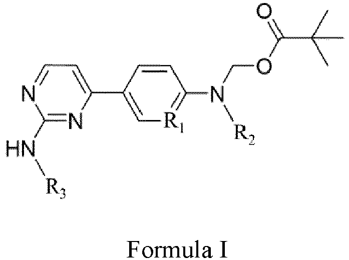 wherein:
R₁ is CorN;
R₂ is selected from 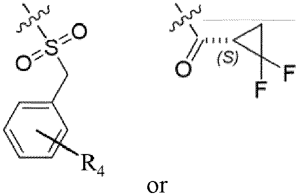 , wherein R₄ is selected from halogen or a cyano group; and
R₃ is selected from a five- or six-membered aryl or heteroaryl group which is unsubstituted or optionally substituted by at least one of halogen, a C₁-C₃ alkyl group, a hydroxyl group, a C₁-C₃ hydroxyalkyl group, an amino group, an amide group and a C₁-C₃ alkylamide group.

2. The JAK inhibitor of claim 1, wherein R₄ is selected from fluorine or a cyano group.

3. The JAK inhibitor of claim 1, wherein the five- or six-membered aryl or heteroaryl group is selected from a phenyl group, a pyridyl group, a pyrimidyl group, a pyrazolyl group, a pyrrolyl group, an imidazolyl group, a pyrazinyl group or a pyridazinyl group.

4. The JAK inhibitor of claim 1, wherein the C₁-C₃ alkyl group is a methyl group, an ethyl group or a propyl group; the C₁-C₃ hydroxyalkyl group is a hydroxymethyl group, a hydroxyethyl group or a hydroxypropyl group; and the C₁-C₃ alkylamide group is a methylformamide group, a dimethylformamide group, an ethylformamide group or a methylacetamide group.

5. The JAK inhibitor of claim 1, wherein R₃ is selected from any one of:

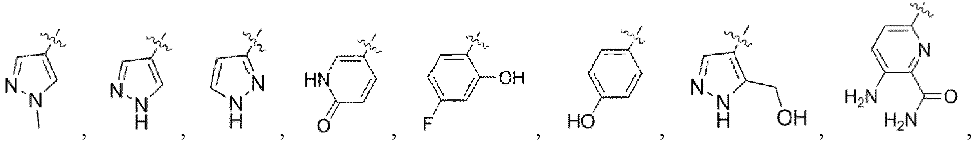

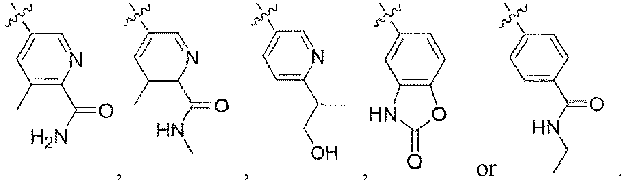

6. Use of the compound of Formula I, or the stereoisomer, geometric isomer, tautomer, hydrate, solvate, or pharmaceutically acceptable salt thereof in preparation of a medication for prevention or treatment of an autoimmune disease or a related inflammatory skin disease.

7. The use of claim 6, wherein the autoimmune disease is at least one of rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, and uveitis.

8. The use of claim 6, wherein the related inflammatory skin disease is at least one of psoriasis, autoimmune-related vasculitis, scleroderma, dermatomyositis, acrodermatitis enteropathica, hidradenitis suppurativa, lichen planus, vitiligo, cutaneous lupus erythematosus, and lichen sclerosus et atrophicus.

9. The use of claim 6, wherein the medication is an orally administered medication.

10. The use of claim 9, wherein the orally administered medication is in a form of a tablet, a pill, a granule, a capsule, a lozenge or a liquid formulation.
